# EUROPEAN PATENT SPECIFICATION

(11) **EP 3 641 820 B1**
(45) Date of publication and mention of the grant of the patent: **28.12.2022**
(21) Application number: 18753256.9
(22) Date of filing: 20.06.2018
(51) Int. Cl.: A61K 31/415, A61K 31/47, A61K 31/496, A61K 45/06, A61P 25/00, A61P 25/16, A61P 25/14, A61P 25/28, A61P 27/02

(54) **COMPOSITIONS COMPRISING AN ANTI-INFLAMMATORY DRUG AND A DICER ACTIVATOR FOR USE IN THE TREATMENT OF NEURONAL DISEASES**
ZUSAMMENSETZUNGEN MIT EINEM ENTZÜNDUNGSHEMMER UND EINEM DICER-AKTIVATOR ZUR VERWENDUNG BEI DER BEHANDLUNG VON NERVENERKRANKUNGEN
COMPOSITIONS COMPRENANT UN MÉDICAMENT ANTI-INFLAMMATOIRE ET UN ACTIVATEUR DICER DESTINÉES À ÊTRE UTILISÉES DANS LE TRAITEMENT DE MALADIES NEURONALES

(30) Priority: 20.06.2017 US 201762522157 P
(43) Date of publication of application: 29.04.2020
(73) Proprietor: Neurosense Therapeutics Ltd., 4676670 Herzeliya (IL)
(72) Inventor: BEN-NOON, Alon, 4720807 Ramat HaSharon (IL)
(74) Representative: Schulz Junghans Patentanwälte PartGmbB
(86) International application number: PCT/IL2018/050684
(87) International publication number: WO 2018/235082

(56) References cited:
- WO-A1-2013/139861
- WO-A1-2014/020608
- US-A1- 2006 183 698
- US-A1- 2015 050 345
- US-A1- 2015 086 616
- YOSHINO T ET AL: "Celecoxib does not induce convulsions nor does it affect GABA"A receptor binding activity in the presence of new quinolones in mice", EUROPEAN JOURNAL OF PHARMACO, ELSEVIER SCIENCE, NL, vol. 507, no. 1-3, 10 January 2005 (2005-01-10), pages 69-76, XP004712258, ISSN: 0014-2999, DOI: 10.1016/J.EJPHAR.2004.11.038
- KLEGERIS A ET AL: "Non-steroidal anti-inflammatory drugs (NSAIDs) and other anti-inflammatory agents in the treatment of neurodegenerative disease", CURRENT ALZHEIMER RESE, BENTHAM SCIENCE PUBL. LTD, NL, vol. 2, no. 3, 1 January 2005 (2005-01-01) , pages 355-365, XP009128606, ISSN: 1567-2050, DOI: 10.2174/1567205054367883
- MERIT E. CUDKOWICZ ET AL: "Trial of celecoxib in amyotrophic lateral sclerosis", ANNALS OF NEUROLOGY., vol. 60, no. 1, 1 July 2006 (2006-07-01), pages 22-31, XP055507408, BOSTON, US ISSN: 0364-5134, DOI: 10.1002/ana.20903 cited in the application
- MAHTAB ASADABADI ET AL: "Celecoxib as adjunctive treatment to risperidone in children with autistic disorder: a randomized, double-blind, placebo-controlled trial", PSYCHOPHARMACOLOGY, SPRINGER, BERLIN, DE, vol. 225, no. 1, 11 July 2012 (2012-07-11) , pages 51-59, XP035158543, ISSN: 1432-2072, DOI: 10.1007/S00213-012-2796-8
- ANIRUDDHA AMRITE ET AL: "Delivery of celecoxib for treating diseases of the eye: influence of pigment and diabetes", EXPERT OPINION ON DRUG DELIVERY, vol. 7, no. 5, 7 March 2010 (2010-03-07), pages 631-645, XP055273549, GB ISSN: 1742-5247, DOI: 10.1517/17425241003663236

## Description

### FIELD OF THE INVENTION

The present invention pertains to compositions, means, and kits for treating neuronal clinical indications in a mammalian subject. The composition comprises a synergistic combination of an anti-inflammatory drug which is celecoxib, and a DICER activator which is ciprofloxacin. Specifically, the present invention pertains to a neuronal disease which is amyotrophic lateral sclerosis (ALS).

### BACKGROUND OF THE INVENTION

Motor neuron diseases (MNDs) are an etiologically heterogeneous group of disorders that are characterized by muscle weakness and/or spastic paralysis, which results from the selective degeneration of lower motor neurons and/or upper motor neurons, respectively.

ALS is a neurodegenerative disease marked by neurodegeneration of both upper and lower motor neurons and progressive muscle impairment, atrophy and death within approximately five years from diagnosis.

Clinically indistinguishable forms of ALS occur as sporadic disease in the absence of known mutation, or can be initiated by genetic mutations. About two-third of familial cases are triggered by mutations of four genes that are chromosome 9 open reading frame 72 (C9ORF72), Cu/Zn superoxide dismutase (SOD1), fused in sarcoma/translocated in liposarcoma (FUS/TLS), TAR-DNA binding protein 43 (TDP43), *see* Volonté, C et a.. CNS & Neurological Disorders-Drug Targets (Formerly Current Drug Targets-CNS & Neurological Disorders), 14(2), 194-207*.*

The etiology of ALS is still not clear. Some etiological factors include involvement of inflammation, and of MicroRNAs, as detailed in the following sections.

Inflammation: Animal and pathological studies suggest that inflammation may contribute to ALS pathology and that non-steroidal anti-inflammatory drugs (NSAIDs) might be protective A typical characteristic of ALS is neuroinflammation. Neuroinflammation is promoted by cyclooxygenase-2 (COX-2), and the activity of COX-2 can be inhibited by non-steroidal anti-inflammatory drugs (NSAIDs). NSAIDs have prolonged survival in transgenic mouse models of ALS, but results from a clinical trial did not find a protective effect of the selective COX-2 inhibitor Celecoxib on ALS disease progression (see Cudkowicz ME, et al (2006). Trial of celecoxib in amyotrophic lateral sclerosis. Ann Neurol.; 60:22-31). Additionally, assessing whether NSAID use before symptom onset can reduce risk or delay the onset of ALS, by a case-control study on NSAID use and ALS (n=111 cases) yielded inconclusive results, see Popat RA, et al. (2007), Amyotroph Lateral Scler.;8:157-63*, and see* Fondell, Elinor et al. Amtyotrophic lateral sclerosis official publication of the World Federation of Neurology Research Group on Motor Neuron Diseases 13.6 (2012): 573-579*.*

MicroRNAs: The cause of ALS is not known, as is the reason why it affects some people and not others. However expert consensus is that molecular alterations in different cells are involved in the development and progression of the disease. For example, motor neuron death is caused by a variety of cellular defects, including the processing of RNA molecules.

During normal aging or neurodegenerative diseases, neuronal survival and function depend on protein homeostasis, which is regulated by multiple mechanisms, including the microRNA (miRNA) pathway. MicroRNAs are a subset of endogenous, small, non-coding RNA molecules involved in the post-transcriptional regulation of eukaryotic gene expression. Produced as long primary transcripts, they are exported to the cytoplasm and further modified to obtain the mature miRNAs, with each step of their biogenesis being a potential step of regulation. Dysregulation in miRNA-related pathways in the central nervous system (CNS) is associated with severe neuronal injury and cell death, which can lead to the development of neurodegenerative disorders, such as amyotrophic lateral sclerosis (ALS), see Rinchetti, P., Rizzuti, M., Faravelli, 1., & Corti, S. (2017). MicroRNA metabolism and Dysregulation in amyotrophic lateral sclerosis. Molecular neurobiology, 1-14*.*

Furthermore, in different cells types, the absence of DICER, a key miRNA processing enzyme, leads to neurodegeneration through cell-autonomous and non-cell-autonomous mechanisms. Loss of certain miRNAs also causes neurodegeneration in some model organisms. On the other hand, miRNA expression is misregulated in patients with different neurodegenerative diseases. Thus, the miRNA pathway appears to be essential in the pathogenesis of several age-dependent neurodegenerative conditions; however, our understanding of the underlying mechanism remains rudimentary. Gascon, E., & Gao, F.-B. (2012). Cause or Effect: Misregulation of microRNA Pathways in Neurodegeneration. Frontiers in Neuroscience, 6, 48*.*

Loss of miRNA biogenesis has been shown to cause spinal motor neuron degeneration *in vivo* (H see Haramati, Sharon, et al. "miRNA malfunction causes spinal motor neuron disease." Proceedings of the National Academy of Sciences 107.29 (2010): 13111-13116*).* Additionally, it was demonstrated that reduction in miRNA levels is a common molecular denominator for multiple forms of familial and sporadic human ALS and that enhancement of DICER activity by a small molecule, enoxacin, is beneficial *in vivo* in two independent ALS mouse models. See Emde, Anna et al. "Dysregulated miRNA Biogenesis Downstream of Cellular Stress and ALS - causing Mutations: A New Mechanism for ALS." The EMBO Journal 34.21 (2015): 2633-2651*.* The rarity of the ALS disease, along with the significant inter- and intra-patient variability in clinical course and a lack of reliable biomarkers, have rendered the development of effective agents to treat ALS a challenge. The need for a safe and effective treatment for ALS is still unmet.

WO2014/020608A1 discloses a method of treating motor neuron disease (MND), comprising administering a therapeutically effective amount of an agent capable of enhancing processing of a pre-miRNA.

### SUMMARY OF THE INVENTION

The references to methods of treatment in this description are to be interpreted as references to the compounds, pharmaceutical compositions and medicaments of the present invention for use in a method for treatment of the human (or animal) body by therapy (or for diagnosis).

The present invention relates to methods and compositions of an anti-inflammatory drug and a DICER activator for treatment of amyotrophic lateral sclerosis (ALS).

As used herein after, the term **"clinical indications"** generally refers hereinafter to neuronal diseases.

As used herein after, the term **"neuronal diseases"** generally refers hereinafter to Motor neuron diseases (MNDs), FTD (Frontotemporal Dementia), macular degeneration (AMD), Alzheimer's disease, Parkinson's disease and autism.

As used herein after, the term "**motor neuron diseases**" or "**MNDs**" generally refers hereinafter to a group of diseases comprising, *inter alia,* amyotrophic lateral sclerosis (ALS), hereditary spastic paraplegia (HSP), primary lateral sclerosis (PLS), spinal muscular atrophy (SMA), spinal bulbar muscular atrophy (SBMA) and lethal congenital contracture syndrome (LCCS).

As used herein after, the term **"anti-inflammatory drugs"** "generally refers hereinafter, in a non-limiting matter, to a group of drugs comprising, *inter alia* steroids, corticosteroids or non-steroidal anti-inflammatory drugs (NSAIDs).

As used herein after, the term **"Cyclooxygenase"** or **"COX",** generally refers hereinafter to an enzyme (specifically, a family of isozymes) that is responsible for formation of prostanoids, including thromboxane and prostaglandins such as prostacyclin, from arachidonic acid. The main COX inhibitors are the non-steroidal anti-inflammatory drugs (NSAIDs).

As used herein after, the term **"low dose"** refers to a therapeutically effective dose of an anti-inflammatory drug, which dose is less than the usual or the conventional dose required to produce the therapeutic effect.

The classical COX inhibitors are not selective and inhibit all types of COX. The resulting inhibition of prostaglandin and thromboxane synthesis has the effect of reduced inflammation, as well as antipyretic, antithrombotic and analgesic effects.

As used herein after, the term **"non-steroidal anti-inflammatory drug"** or **"NSAID"** refers hereinafter to celecoxib.

As used herein after, the term **"DICER"** generally refers hereinafter to an enzyme that cleaves double-stranded RNA (dsRNA) and pre-microRNA (pre-miRNA) into short double-stranded RNA fragments called small interfering RNA and microRNA, respectively. These fragments are approximately 20-25 base pairs long with a two-base overhang on the 3' end. Dicer facilitates the activation of the RNA-induced silencing complex (RISC), which is essential for RNA interference. RISC has a catalytic component argonaute, which is an endonuclease capable of degrading messenger RNA (mRNA).

It is one object of the invention to disclose a composition for use in treating amyotrophic lateral sclerosis (ALS) in a mammalian subject, wherein the composition comprises a synergistic combination of celecoxib and ciprofloxacin, wherein the celecoxib to ciprofloxacin ratio ranges from 1:10 to 1:200 (wt/wt).

It is another object of the invention to disclose the composition for use as defined above wherein the ciprofloxacin is ciprofloxacin-HCI.

It is another object of the invention to disclose the composition for use as defined in any of the above wherein the anti-inflammatory drug is administered in a dose lower than indicated for anti-inflammatory clinical effect.

It is another object of the invention to disclose the composition as defined in any of the above wherein the composition is configured to be administrable in a manner selected from a group consisting of a tablet, a capsule, a pill, lyophilized, powder, emulsion, granulated powder, cream, ointment, paste, lotion gel, liquid, a solution, a patch and any combination thereof.

It is another object of the invention to disclose the composition as defined in any of the above wherein the composition is configured to be administrable in a manner selected from a group consisting of fast release, slow release, sustained release, controlled release and any combination thereof.

It is another object of the invention to disclose the composition as defined in any of the above wherein the composition additionally comprising ingredients selected from a group consisting solubilizers, stabilizers, buffers, tonicity modifiers, bulking agents, viscosity enhancers/reducers, surfactants, chelating agents, adjuvants and any combination thereof.

It is one object of the invention to disclose a kit comprising celecoxib and ciprofloxacin for use in a synergistic treatment of amyotrophic lateral sclerosis (ALS) in a mammalian subject, wherein the celecoxib to ciprofloxacin ratio ranges from 1:10 to 1:200 (wt/wt).

It is another object of the invention to disclose the composition for use as defined in any of the above wherein the composition is configured to be administrable in a manner selected from a group consisting of oral, topical, dermal, transdermal, intravenous, subcutaneous, intramuscular, intra-articular, suppository, intraventricular, inhalational, aerosol, sublingual and any combination thereof.

It is another object of the invention to disclose the composition for use as defined in any of the above wherein the composition is configured to be administrable in a manner selected from a group consisting of one at a time, non-simultaneous, sequentially and concomitantly.

It is another object of the invention to disclose the composition for use as defined in any of the above wherein the composition is configured to be administrable in a manner selected from a group consisting of single dose, single daily dose, twice daily dose, continuous dose, infusion, and any combination thereof.

### BRIEF DESCRIPTION OF THE DRAWINGS

The present invention will hereinafter be described in conjunction with the following drawing figures:
**Figure 1****:** Scheme exhibiting the treatment protocol;
**Figure 2****:** The change in distance moved (in %) of non- treated SOD1 G93R compared to wild type and to the treated SOD1 larva in the recovery from challenge phase;
**Figure 3****:** Ciprofloxacin and Celecoxib combinations - treated SOD1 mutants' locomotor activity compared to non- treated SOD1 mutants in all 3 phases of the experiment;
**Figure 4****:** Ciprofloxacin and Celecoxib combinations- treated SOD1 mutants' locomotor activity compared to non- treated SOD1 mutants during the whole period of measurement;
**Figure 5****:** The distance moved measured and calculated for 96 SOD1 mutant vs WT larvae per time bin of 1 minute;
**Figure 6****:** SOD1 mutants displayed a significant reduction in their locomotor activity compared to WT during the whole period of measurement;
**Figure 7****:** SOD1 mutants displayed a significant reduction in their locomotor activity compared to WT in all 3 phases of the experiment;
**Figure 8****:** Riluzole- treated SOD1 mutants displayed a significant elevation in their locomotor activity compared to non-treated Sod1 mutants in all 3 phases of the experiment;
**Figure 9****:** Celecoxib- treated SOD1 mutants' locomotor activity compared to non- treated SOD1 mutants in all 3 phases of the experiment;
**Figure 10****:** Ciprofloxacin- treated SOD1 mutants' locomotor activity compared to non- treated SOD1 mutants in all 3 phases of the experiment;
**Figure 11****:** Summary of locomotor activity of SOD1 mutants treated with Ciprofloxacin distinct concentrations during all 3 phases of the experiment;
**Figure 12****:** Enoxacin- treated SOD1 mutants' locomotor activity compared to non- treated SOD1 mutants in all 3 phases of the experiment;
**Figure 13****:** Normalized enoxacin vs Ciprofloxacin- treated SOD1 mutants' locomotor activity compared to non- treated SOD1 mutants in all 3 phases of the experiment;

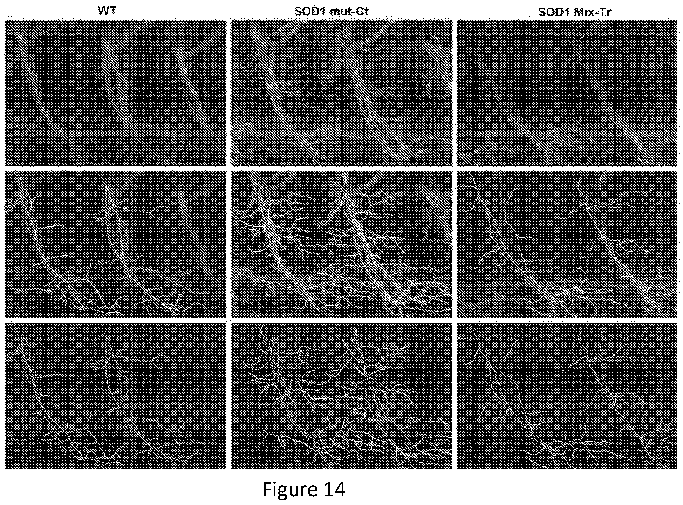

**Figure 14****:** Administration of Ciprofloxacin and Celecoxib recovered axonopathy of SOD1 mutant fish. Morphological analysis of individual motor neurons in the trunk of zebrafish larva; segments S10- S12 were used for analysis. Left panel- WT, middle panel- non-treated SOD1 mutant fish, left panel- 1µM Celecoxib + 100µM Ciprofloxacin- treated SOD1 mutant fish. Upper panel- 3D reconstructed apotome z-stack images of branching motor neurons in the trunk of 6dpf zebrafish larvae immunostained with anti-acetylated tubulin antibodies. Middle and lower panels- The backbone (colored processes) of motor neurons traced with the Filaments analysis of Imaris software (Bitplane; in yellow- single motor neuron backbone). n=15; control and treated SOD1 mutants and n=11; WT fish; and
**Figure 15A****,** **15B****,** **15C****:** Combination of Ciprofloxacin and Celecoxib caused a nearly full recovery of motor neurons axonopathy of SOD1 mutant fish. Aspects of length and branching of motor neurons axonal projections were calculated using the Imaris software (Bitplane) and are plotted in the graphs.

### DETAILED DESCRIPTION OF PREFERRED EMBODIMENTS

The present invention discloses a composition for use in treating amyotrophic lateral sclerosis (ALS) in a mammalian subject, wherein the composition comprises a combination of celecoxib and ciprofloxacin, wherein the celecoxib to ciprofloxacin ratio ranges from 1:10 to 1:200 (wt/wt).

The present invention discloses therapeutic compositions and therapeutic uses thereof, for MNDs, specifically ALS, aiming to present an effective treatment for patients diagnosed with an MND, specifically ALS. The technology hereto presented underlines the role of miRNAs in addition to anti-inflammatory drug which has a neuroprotective effect in the development of ALS by using *in vivo* and *in vitro* pharmacological models of ALS, see Berthod, François, and François Gros-Louis. "In vivo and in vitro models to study amyotrophic lateral sclerosis. " Amyotrophic Lateral Sclerosis. InTech, 2012.

In a non-claimed aspect, the present description discloses a combination of a quinolone, which is a DICER enhancer (specifically ciprofloxacin), and an anti-inflammatory agent (specifically celecoxib), as a treatment.

Ciprofloxacin is a quinolone, specifically a fluoroquinolone, which enhances Dicer activity and is therefore able to upregulate microRNA biogenesis, and celecoxib as a COX-2 selective nonsteroidal anti-inflammatory drug (NSAID).

The quinolones are broad-spectrum antibacterial agents that have a novel mechanism of action. As synthetic compounds, these agents have been developed extensively to optimize antimicrobial activity, pharmacokinetic properties, and drug safety. The quinolones have wide potential applications and a broader spectrum of activity. Ciprofloxacin remains the most potent quinolone against *Pseudomonas aeruginosa,* see Walker, R. C. (1999, October). The fluoroquinolones. In Mayo Clinic Proceedings (Vol. 74, No. 10, pp. 1030-1037*).*

Quinolones cause synergistic or additive toxicity with NSAIDs. The concomitant administration of quinolones and nonsteroidal NSAIDs has reportedly led to increased CNS stimulation. The concomitant administration of quinolones and NSAIDs has been reported to increase the risk of CNS stimulation and convulsive seizures. Patients with CNS disorders or other risk factors that may predispose them to seizure development or patients taking drugs that lower the seizure threshold may not be appropriate candidates for NSAID usage if they are also taking a quinolone. Use a quinolone with caution in individuals who take a NSAID concomitantly.

As a consequence, the aforesaid teaching against prior art is hereby contradicted, for the present invention's novel claim that the combination of the two suggested drugs results in a positive, significant and surprising outcome to ALS patients, which don't have risk factors that may predispose them to seizure development, perhaps with a new formulation that extends the release of the drugs for a continues treatment.

Specifically, epileptogenic activity induced by combined treatment with anti-inflammatory drugs and enoxacin was investigated in chronic electrode-implanted rats. Ferubinac ethyl and aspirin DL-lysine showed a spike and wave complex in EEG without showing remarkable behavioral changes when they were injected intraventricularly, although a relatively high dose was needed. Enoxacin, on the other hand, elicited potent epileptogenic activity characterized by uninterrupted high voltage spike and wave complex at doses of 50 and 100 micrograms. At the same time, rats showed hyperactivity, jumping and violent convulsion. Combined treatment with enoxacin (p.o.) and ferubinac ethyl (i.v.) caused potent epileptogenic activity characterized by uninterrupted burst of high voltage spike and wave complex. Behaviorally, animals showed forelimb clonus, head nodding and generalized convulsion. High voltage spike and wave complex was also observed after combined treatment with enoxacin (i. vent.) and ferubinac ethyl (i.v. or i. vent.) in association with hyperactivity and jumping and violent convulsion.). It is concluded that simultaneous treatment with enoxacin and ferubinac ethyl produced epileptogenic activity when injected intraventricularly, see Kamei, Chiaki, et al. "Epileptogenic activity induced by combined treatment with antiinflammatory drugs and enoxacin and its inhibition by a calcium antagonist, nicardipine. "Methods and findings in experimental and clinical pharmacology 18.9 (1996): 579-588*.*

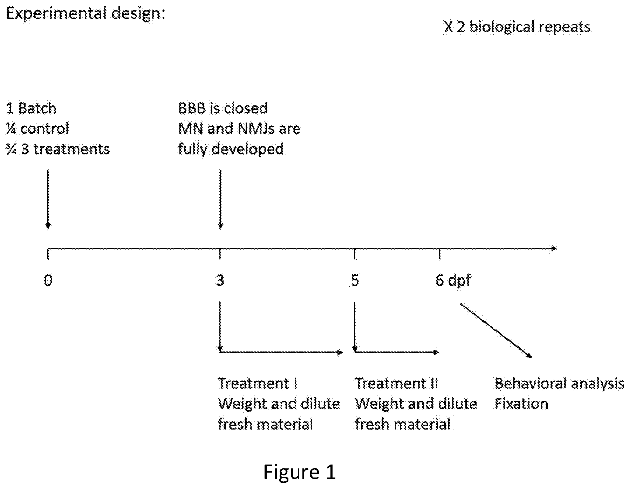

The present invention used SOD1 G93R mutant and WT fish to assess the effect of anti-inflammatory agents and DICER activators compared to available treatments for ALS,(see Figure 1 for treatment protocol).

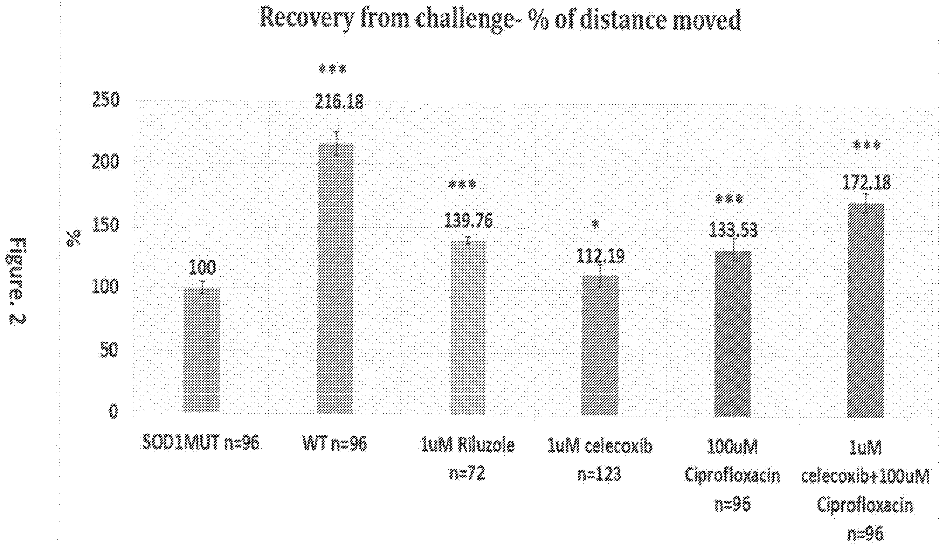

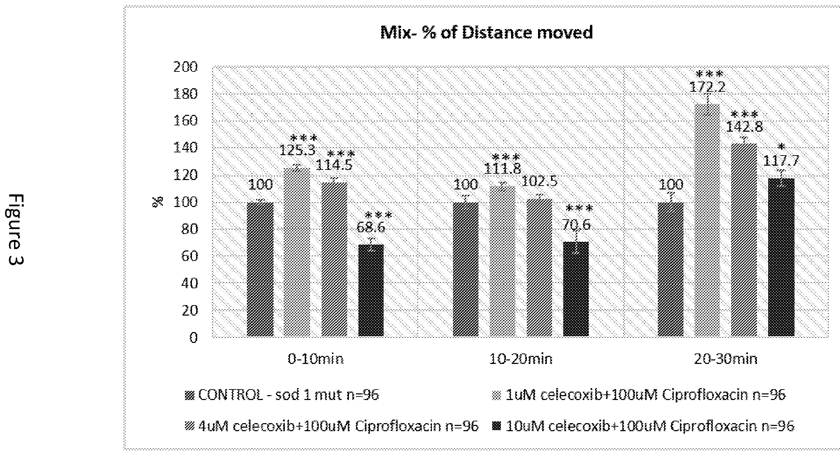

The present invention recites **a synergistic effect** in SOD1 mutant fish treated with both Ciprofloxacin and Celecoxib, specifically at concentrations of 1µM Celecoxib and 100µM Ciprofloxacin. At these concentrations, there was a dramatic effect on their swimming ability compared to non- treated mutants **(****Figures 2****, 3).**

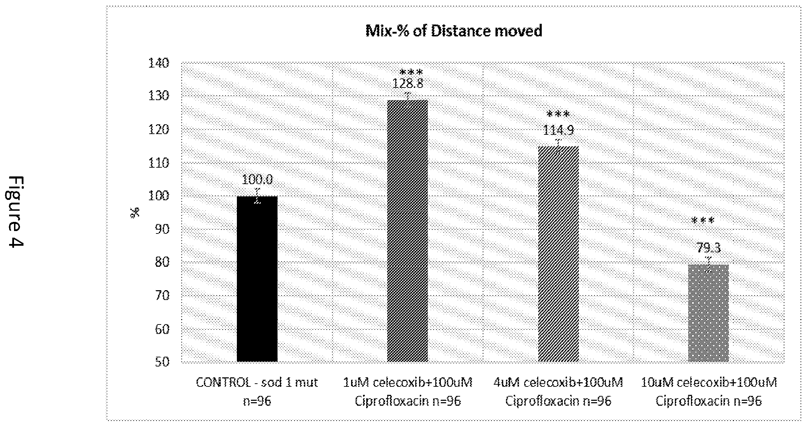

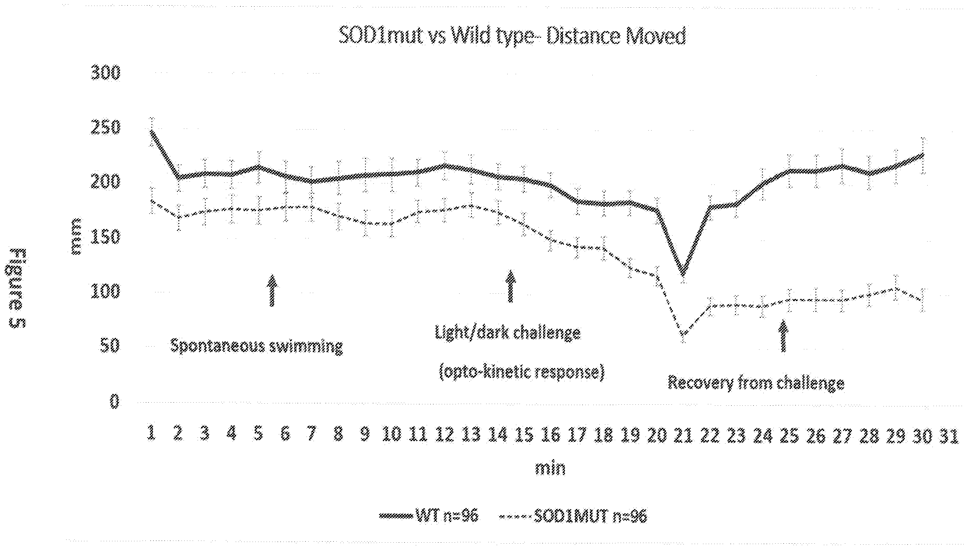

The treated SOD1 mutant larva distance moved was elevated by 29% compared to non- treated SOD1 mutant swimming behavior **(****Figure 4****).** The effect was striking enough that the treated SOD1 fish behavior closely resembled that of wild type fish **(****Figure 5****).**

The combination of the present invention is in a ratio ranging from 1:10 to 1:200 (wt/wt), of an anti-inflammatory drug, which is celecoxib, and a DICER activator, which is ciprofloxacin.

### EXAMPLE 1

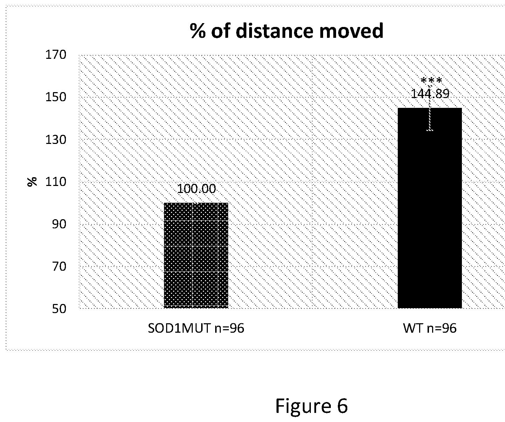

Transgenic zebrafish for the top ALS-linked gene superoxide dismutase 1 (SOD1) were utilized in this study. It was previously shown that the SOD1 G93R mutant fish recapitulate the major phenotypes of ALS including neuromuscular junction defects, decreased endurance, motor neurons loss and muscle pathology, see Ramesh T, Lyon AN, Pineda RH, et al. A genetic model of amyotrophic lateral sclerosis in zebrafish displays phenotypic hallmarks of motoneuron disease. Disease models & mechanisms 2010; 3:652-62*.* SOD1 mutation caused behavioral deficits related to locomotion. The SOD1 mutant animals showed significant reduction in their swimming ability compared to the wild type during the spontaneous swimming, light/ dark challenge and most dramatically following the second peak of stress, exhibiting recovery from the challenge **(****Figures 5-7****).** The distance the wild type larva moved was averaged for the whole period of time and was elevated by 44% compared to SOD1 mutant swimming behavior **(****Figure 6****).**

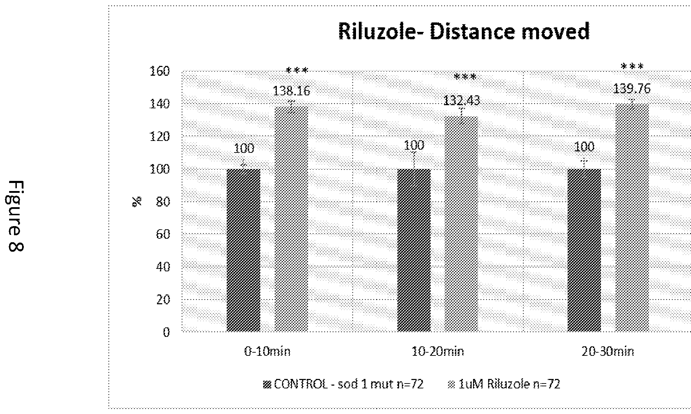

As a positive control, the SOD1 mutant fish were treated with Riluzole **(****Figure 8****).** Riluzole was chosen for this study as it was for years the only established drug shown to have a disease-modifying effect in ALS patients, see Bensimon G, Lacomblez L, Meininger V, Group tARS. A Controlled Trial of Riluzole in Amyotrophic Lateral Sclerosis. New England Journal of Medicine 1994;330:585-91*.* The SOD1 mutant animals treated with Riluzole showed significant elevation of 36% compared to SOD1 mutant swimming behavior.

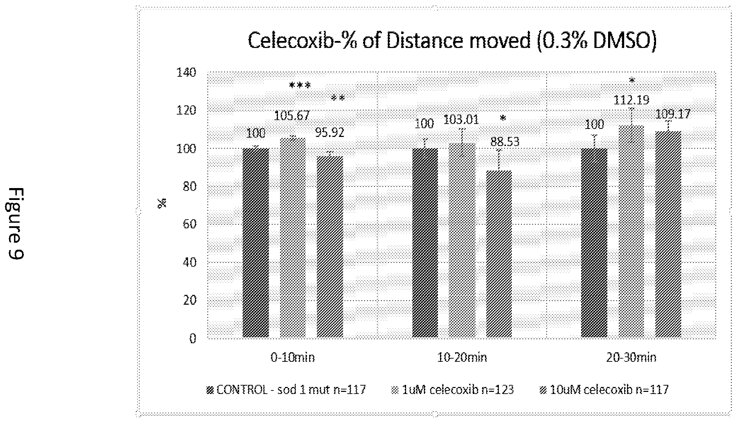

In the next series of experiments, SOD1 mutant fish were treated with Celecoxib at distinct concentrations **(****Figure 9****).** Toxicity of Celecoxib was noticeable in high concentrations (30µM) in both SOD1 mutants and WT animals, including cardiovascular toxicity and death. Treatment of SOD1 mutant larvae with lower Celecoxib concentrations revealed subtle locomotor decline in 10µM (with 0.3% DMSO background) and in 5µM (with 0.1% DMSO as a background). Below these concentrations, no toxicity was evident and the overall morphology and behavior were normal Importantly, our results show that Celecoxib toxicity is parallel in both ALS and healthy subjects and the SOD1 mutants do not show higher susceptibility to the drug (section III). Overall, treatment at all doses did not strikingly improve swimming behavior. At 1µM, there was a 6% increase in swimming activity as compared to untreated SOD1 mutant swimming behavior.

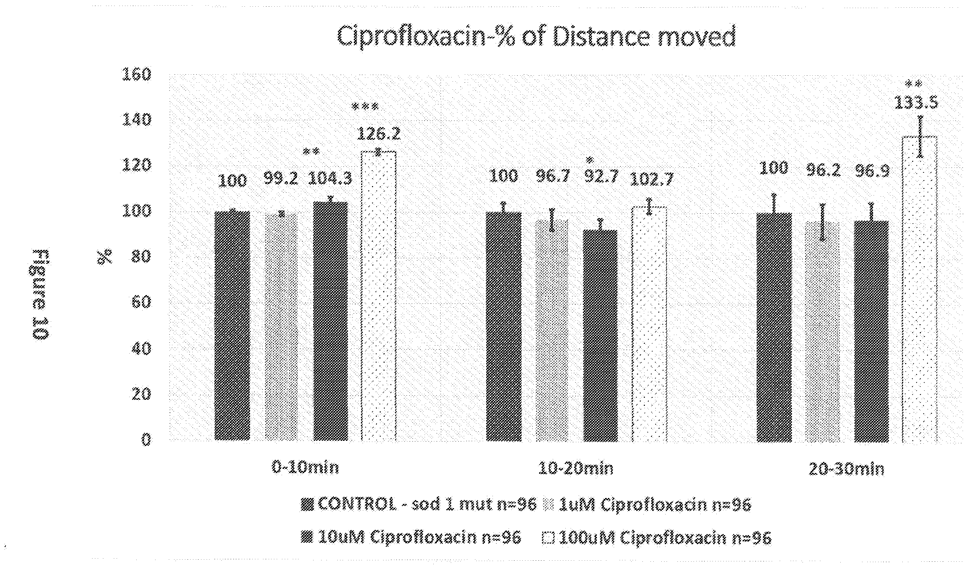

SOD1 mutant fish were then treated with Ciprofloxacin at concentrations of 500, 200, 100, 50, 10 and 1µM **(****Figures 10****, 14).** All Ciprofloxacin doses were safe for the fish, showing no overall or subtle morphological and behavioral toxicity. The higher concentrations had a positive effect on SOD1 mutants' swimming ability with the 100µM concentration showing the greatest benefit. At that level, SOD1 mutant larva distance moved was elevated by 18% compared to non- treated SOD1 mutant swimming behavior).

SOD1 mutant fish treated with Ciprofloxacin showed similarity to treatment with the same doses of Enoxacin, its family member **(****Figure 10****).** Enoxacin- treated SOD1 mutant larva distance moved was elevated by 20% compared to non- treated SOD1 mutant swimming behavior.

Next, SOD1 mutant fish were treated with both Ciprofloxacin and Celecoxib at a variety of concentrations. The greatest effect was seen at concentrations of lµM Celecoxib and 100µM Ciprofloxacin; at these concentrations, there was a dramatic effect on their swimming ability compared to non- treated mutants **(****Figure 3****).** The treated SOD1 mutant larva distance moved was elevated by 29% compared to non- treated SOD1 mutant swimming behavior **(****Figure 4****).** The effect was striking enough that the treated SOD1 fish behavior closely resembled that of wild type fish.

To summarize the distinct effects of the treatments on fish motor abilities, we averaged the last 10 minutes of the distance moved **(****Figure 2****).** Following introduction of additional muscle and neurological stress, this recovery from challenge phase is aimed to identify enhanced muscle endurance. Wild type animals swam longer distances during this period compared to the SOD1 mutant fish (elevation of 116%). Riluzole induced a clear increase in the swimming abilities of the fish (39.7%), but significant synergistic effect was seen with the combination of Ciprofloxacin at 100µM and celecoxib at 1µM (72%, **Figure 2). Figure 2** discloses the change in distance moved (in %) of non- treated SOD1 G93R compared to wild type and to the treated SOD1 larva in the recovery from challenge phase.

Following these compelling locomotor activity assays results, we conducted morphological assays, analyzing motor neurons morphology following the treatment with the combination of Ciprofloxacin at 100µM and Celecoxib at 1µM **(****Figures 14-15****).** WT zebrafish predominantly exhibited normal motor neurons, with long and moderately branched axons, while the SOD1 mutant showed severe axonopathy with highly complex branched fibers. When treated with the combination of 1µM Celecoxib and 100µM Ciprofloxacin, SOD1 mutant larvae showed significant recovery of the mutant morphology, and gained nearly normal axon morphology **(****Figures 14-15****).** Remarkably, combination of Ciprofloxacin and Celecoxib caused a nearly full recovery of motor neurons axonopathy of SOD1 mutant fish.

This combination of Ciprofloxacin and Celecoxib, as indicated from these pre- clinical studies, may impact neuropathology and degeneration in ALS patients, slowing or inhibiting the disease.

### EXAMPLE 2

### a. Methodology

Fish - Adult and larval zebrafish (Danio rerio) were maintained at the Russek- Blum laboratory's fish facility (Dead Sea & Arava Science Center; Yair station, Central Arava) at 28.5°C and bred according to established procedures²⁵. Animal protocols were approved by the Ben Gurion University Committee on Use and Care of Animals. Tg(sodl:sod1G93R or WT; hsp70:DsRed) transgenic lines were used in this study, see Ramesh T, Lyon AN, Pineda RH, et al. A genetic model of amyotrophic lateral sclerosis in zebrafish displays phenotypic hallmarks of motoneuron disease. Disease models & mechanisms 2010; 3:652-62*.* To generate zebrafish expressing transgenic mutant Sod1, a zebrafish genomic region containing the endogenous sodl promoter and sodl gene was used. Sodl was mutated by changing glycine 93 to arginine (G93R); this mutation affects a conserved amino acid that is often mutated in familial ALS, see Orrell R, de Belleroche J, Marklund S, Bowe F, Hallewell R. A novel SOD mutant and ALS. Nature 1995;374:504*, and* Elshafey A, Lanyon WG, Connor JM. Identification of a new missense point mutation in exon 4 of the Cu/Zn superoxide dismutase (SOD-1) gene in a family with amyotrophic lateral sclerosis. Human molecular genetics 1994;3:363-4*.*

Materials and solubility- **Celecoxib** To achieve high concentration of Celecoxib (Celecoxib BP/EP; batch no. CLX/008/04/17; 25gr; Prudence Pharma Chem, India), the powder was dissolved to a stock solution of 100mM in 100% DMSO. The powder was completely dissolved in 100% DMSO, but while diluting the 100µM Celecoxib stock into final concentration of 100uM (1:1000) in zebrafish raising buffer (DB), the material precipitated, creating particles and clouds. Celecoxib was then dissolved to a stock solution of 10µM in 100% DMSO. The powder was completely dissolved in 100% DMSO, and when diluted to final concentration of 30µM in DB (1:333.3 dilution), the material had stayed in the buffer without crashing. The materials were added to DB in the highest planned concentrations and stored in petri dishes in 28°C incubator, simulating the exact conditions of the experiment. The final DMSO concentration in the 30µM Celecoxib in DB was 0.3%. Inspection of the stock solution showed clear solution with no aggregates seen by the naked eye nor under the microscope. In the final diluted solution in DB, no aggregates were observed also after 24 or 48 hrs. Spectrophotometer absorption was observed at 0, 24 and 48 hours after preparation.

Materials and solubility- **Ciprofloxacin** To achieve high concentration of Ciprofloxacin (Ciprofloxacin hydrochloride Ph,Eur; batch no. CP20517124; 25gr; Neuland Laboratory LTD, India), the powder was dissolved to a stock solution of 100mM in ddH2O. The powder was completely dissolved in ddH2O and completely dissolved when diluted to final concentration of 100uM in DB (1:1000 dilution). The final DMSO concentration in the 100uM Ciprofloxacin in DB was 0.1%. Inspection of the stock solution showed clear yellow solution with no aggregates seen by the naked eye nor under the microscope. In the final diluted solutions in DB, no aggregates were observed also after 24 or 48 hrs. To adjust the pH to the control sample (0.1 % DMSO in DB; pH 6.72), 12ul of 100mM NaOH were added to the 100uM Ciprofloxacin solution (pH 6.32). No addition of NaOH was needed in lower concentration of Ciprofloxacin in DB. Spectrophotometer absorption was analyzed at 0, 24 and 48 hours after preparation **(****Figure 3****).** A light yellow color of the solution caused higher absorption values. Mix of highest concentrations (100uM Ciprofloxacin + 30µM Celecoxib + NaOH) was measured for absorption in spectrophotometer at 0, 24 and 48 hours after preparation.

Thirty (30) uM Celecoxib had a different absorption compared to the control (DB) of 0.017, 0.013 and 0.021 at 0, 24 and 48 hours after preparation, respectively. 100uM Ciprofloxacin gave differences in absorption compared to the control of 0.123, 0.149 and 0.155 at 0, 24 and 48 hours after preparation, respectively, due to the clear, light yellow color of the solution. Mix of highest concentrations (100uM Ciprofloxacin + 30µM Celecoxib) gave differences in absorption compared to the control of 0.16, 0.154 and 0.131 at 0, 24 and 48 hours after preparation, respectively.

Zebrafish raising buffer (DB) used as the basis of all experiments contains final concentrations of: 1.74mM NaCl, 0.21mM KC1, 0.12mM MgSO4, 0.18mM Ca(NO3)2, 0.15mM HEPES in ddH2O, final pH= 6.7.

**Treatment protocol** SOD1 G93R mutant or WT fish were collected and raised according to accepted and approved protocols, see Westerfield M. The Zebrafish Book: a Guide for the *Laboratory Use of Zebrafish (Danio rerio) 2000.* Larvae's morphology was observed (heart rate, overall morphology and behavior) at each day.

**Figure 1** is scheme exhibiting the treatment protocol.

Two-hundred and forty (240) larvae were selected from the same laying batch to be introduced to three distinct treatments and a control (n=60 per each treatment). First and second treatments were conducted at 3 and 5 days post fertilization (dpf), respectively, when the blood brain barrier (BBB) is fully closed, and the motor neurons (MNs) and neuromuscular junctions (NMJs) are fully developed. The materials were administrated in the swimming water (DB). At day 6, larvae were taken to behavioral analysis using the automated DanioVision^{™} system. Following analysis, larvae were fixed and whole- mount immunohistochemistry procedure was conducted to be used for high resolution morphological analysis. Treatment protocol is detailed in **Figure 1****.**

Toxicity analysis Fish were observed during the day of administration and the following days to observe toxicity. Acute toxicity was evaluated for apoptosis/necrosis and cardiovascular system abnormalities (heart rate, morphology, hemorrhage and edema). Head, eyes and overall morphological and behavioral toxicity were recorded according to accepted procedures²⁸.

Behavioral analysis DanioVision^{™}, an automated high-throughput tracking system of zebrafish larvae from Noldus Information Technology (Wageningen, the Netherlands), including control of light and dark conditions was used for the measurements and analysis of the results. Each single larvae was put in a single well, with the same volume of swimming water to ensure uniform background. Each animal was tested for its x,y position using dynamic subtraction 30 times per second. The distance each larvae moved in mm was calculated from the x,y position and averaged per time bin of 1 minute (average of 1800 measurements per each 1 minute). The behavioral profile was measured in 3 phases according to changes in environment that we applied, to introduce additional muscle and neurological stress. Spontaneous swimming, light/ dark challenge (opto-kinetic response) and recovery from challenge behaviors were recorded and analyzed. All animals from the same treatment: wild type (WT), SOD1 mutant non- treated or treated were averaged per time bin of 1 minute to examine their reaction to additional stress, and then calculated for the whole period of time to get a relative number compared to the non- treated SOD1 mutant swimming behavior. Statistical analysis used was paired Student's t-tests (two-tailed) with two-sample assuming unequal variances. All P-values reported are two-tailed and the significance level was set at 0.05 (^{∗}), 0.01 to 0.001 (^{∗∗}) and below 0.001 (^{∗∗∗}).

Morphological analysis Whole-mount staining was performed on 6 dpf treated and non-treated SOD1 G93R or WT using anti- acetylated tubulin antibodies using established immunohistochemistry procedures. Spinal motor neuron axonal projections (segments 10-12) stained with anti- acetylated tubulin were imaged using Zeiss apotome microscopy. 3D reconstructed images were used for quantification using the Imaris image analysis software (Bitplane LTD).

### b. Results

### I. Transgenic SOD1 mutant larvae show impairments in locomotor activity

Larval zebrafish expressing ALS-causing SOD1 G93R mutation were shown previously to have impairments in locomotor activity with abnormal motor axon projections, but otherwise have normal gross morphology, see Lemmens R, Van Hoecke A, Hersmus N, et al. Overexpression of mutant superoxide dismutase 1 causes a motor axonopathy in the zebrafish. Human molecular genetics 2007;16:2359-65*, and* Ramesh T, Lyon AN, Pineda RH, et al. A genetic model of amyotrophic lateral sclerosis in zebrafish displays phenotypic hallmarks of motoneuron disease. Disease models & mechanisms 2010;3:652-62*.*

To verify and elaborate our understanding of motor dysfunction in these larvae, we performed video analyses using the DanioVision system, as described in the materials and methods section. Two biological repeats were conducted, analyzing the locomotor activity of 48 animals in each. **Figure 5** depicts the distance moved measured and calculated for 96 SOD1 mutant vs. larvae per time bin of 1 minute.

The parameter analyzed was the distance the larvae moved (in mm) In the first graph all animals-SODlmut or wild-type (WT) swimming behavior were averaged per time bin of 1 minute to see whether their reaction to additional stress was as expected (**Figure 5**).

In all time points averaged, the distance that the WT larvae swam in mm was significantly higher compared to the SOD1 mutant fish. During spontaneous swimming, following light/ dark challenge and after recovery from challenge (**Figure 5**).

Next, the distances in mm all WT larvae swam in time bin of 1 minute were averaged for the whole period of time to get a relative number compared to the SOD1 mutant swimming behavior (**Figure 6**).

During the whole experiment, the distance in mm that the SOD1 mutant larvae swam was significantly reduced compared to the WT larvae. The WT larvae swam nearly 45% more distance than the SOD1 mutants **(****Figure 6****).**

**Figure 6** discloses that SOD1 mutants displayed a significant reduction in their locomotor activity compared to WT during the whole period of measurement.

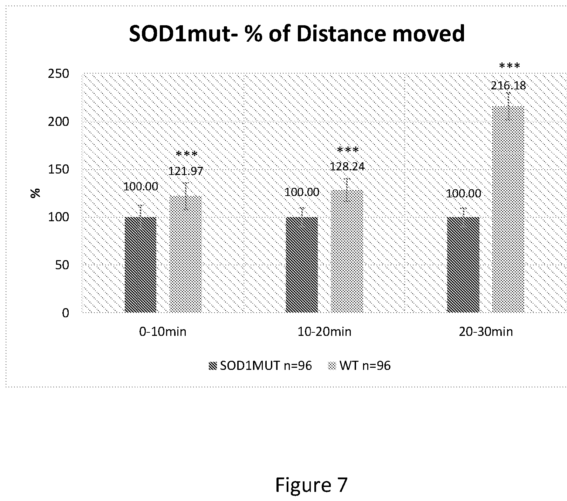

To analyze the locomotor activity in SOD1 mutants compared to the WT during the different phases of the experiment, following the different responses to stress, we calculated the distance moved per each part of the experiment **(****Figure 7). Figure 7** discloses that SOD1 mutants displayed a significant reduction in their locomotor activity compared to WT in all 3 phases of the experiment.

When changes in the swimming environment are applied, additional muscle and neurological stress are introduced. During spontaneous swimming (0- 10 minutes of the experiment) and light/ dark challenge (opto-kinetic response; 10-20 minutes), SOD1 mutants displayed significant reduction in their locomotor activity compared to WT. WT larvae swam 22-28% more than the SOD1 mutants. Following the two peaks of stress (t= 10, 20 minutes), a behavior of recovery from challenge was evident. The larvae froze and then gradually recovered back to values of spontaneous swimming During this phase, SOD1 mutants recovered less efficiently, to a lower locomotor swimming behavior. In this phase, the WT larvae swam 116% more distance than the SOD1 mutants.

### II. Verification- Transgenic SOD1 mutant larvae treated with Riluzole show elevation in locomotor activity

Chronic glutamate excitotoxicity may accumulate to toxic levels and contribute to neuronal death in ALS. This provided a rational basis for undertaking a clinical trial with riluzole, a drug with complex effects, but which appears to block the presynaptic release of glutamate. Riluzole demonstrates a modest increase in survival in treated participants (up to 2-3 months) and delays the onset of ventilator-dependence or tracheostomy in selected patients, see Bensimon G, Lacomblez L, Meininger V, Group tARS. A Controlled Trial of Riluzole in Amyotrophic Lateral Sclerosis. New England Journal of Medicine 1994;330:585-91*.*

To determine whether our zebrafish model has the potential to identify novel ALS therapies, we tested the ability of the antiexcitotoxic drug riluzole to modify neuronal stress in the SOD1 mutant zebrafish larvae. Riluzole was chosen for this study as it was for years the only established drug shown to have a disease-modifying effect in ALS patients and since it is the standard of care.

Riluzole was administrated to the swimming water of the SOD1 mutant larvae in a final concentration of 1µM, a dose that was tested and showed no toxicity in SOD1 mutant zebrafish, see McGown A, Shaw DPJ, Ramesh T. ZNStress: a high-throughput drug screening protocol for identification of compounds modulating neuronal stress in the transgenic mutant sod1G93R zebrafish model of amyotrophic lateral sclerosis. Molecular neurodegeneration 2016;11:56*.*

When SOD1 mutants were treated with this concentration of Riluzole, neuronal stress in inhibitory interneurons was reduced, see McGown A, McDearmid JR, Panagiotaki N, et al. Early interneuron dysfunction in ALS: insights from a mutant sodl zebrafish model. Annals of Neurology 2013; 73:246-58*.*

When treated with Riluzole, the distances that the SOD1 mutant larvae swam in mm were higher compared to the non- treated SOD1 mutant fish during all phases of the experiment.

The distances in mm all Riluzole- treated SOD1 mutant larvae swam in a time bin of 1 minute were averaged for the whole period of time to get a relative number compared to the non-treated SOD1 mutant swimming behavior. Riluzole-treated SOD1 mutants displayed a significant elevation in their locomotor activity compared to non- treated SOD1 mutants during the whole period of measurement.

During the whole experiment, the distance in mm that the Riluzole- treated SOD1 mutant larvae swam was significantly higher (elevation of 36.8%) compared to the non- treated SOD1 mutant larvae.

To analyze the locomotor activity in SOD1 mutants compared to the WT during the different phases of the experiments, and following the different responses to stress, we calculated the distance moved per each part of the experiment (Figure 8).

**Figure 8** discloses that riluzole-treated SOD1 mutants displayed a significant elevation in their locomotor activity compared to non-treated Sodl mutants in all 3 phases of the experiment.

Treatment with riluzole caused constant elevation in locomotor activity in all phases of the experiment. Riluzole-treated SOD1 mutants displayed significant increase in their locomotor activity compared to non- treated SOD1 mutant larvae during spontaneous swimming (38% increase), light/ dark challenge (32% increase) and recovery from challenge (39.7%).

### III. Toxicity and efficacy of treatment with Celecoxib in ALS (SOD1 mut) model

The first material to be tested was the cox2 inhibitor Celecoxib that was suggested to have a role as neuro-inflammatory modulator in maintaining macrophages in their neuroprotective state (see Aid S, Bosetti F. Targeting cyclooxygenases-1 and -2 in neuroinflammation: therapeutic implications. Biochimie 2011;93:46-51*.* Celecoxib was introduced to the swimming water of the SOD1 mutant larvae in three final concentrations, 30µM, 10µM and lµM. Due to solubility constrains, the experiment was conducted with the background of 0.3% DMSO in all samples (including the control sample).

Summary of section III: While using 0.3% DMSO as a background, Celecoxib caused cardiovascular toxicity and death in the concentration of 30µM. Subtle locomotor reduction was evident in 10µM Celecoxib treatment during specific phase of the experiment. Reducing DMSO to 0.1% as a background, caused increase in toxicity produced by Celecoxib. Although the overall morphology and behavior were normal, reduction in locomotor ability was observed below 10µM Celecoxib and was evident at 5µM.

Superoxide dismutase enzyme is a central antioxidant catalyst that uses 02- as a substrate (02-causes a considerable degree of biological damage), reducing its levels into ordinary molecular oxygen (02) or hydrogen peroxide (H202), see Halliwell B, Gutteridge JMC. [1] Role of free radicals and catalytic metal ions in human disease: An overview. Methods in Enzymology: Academic Press; 1990:1-85*. In vivo,* Celecoxib is oxidized by cytochrome P450 (CYP450) 2C9 and 3A4 to the inactive metabolite hydroxycelecoxib, and then hydroxycelecoxib is converted to carboxycelecoxib and celecoxib glucuronide' , see Gong L, Thorn CF, Bertagnolli MM, Grosser T, Altman RB, Klein TE. Celecoxib pathways: pharmacokinetics and phannacodynamics. Pharmacogenetics and Genomics 2012;22:310-8*.* The results showing a shift in toxicity depending on DMSO levels can be explained by a possible hypothesis suggesting a role of DMSO, a powerful scavenger of •OH in protection from Celecoxib toxicity in SOD1 mutants.

Nevertheless, no substantial efficacy was evident using all concentrations of Celecoxib.

### IV: Toxicity and efficacy of treatment with Ciprofloxacin in ALS (SOD1 mut) model

The second material to be tested was the Ciprofloxacin previously suggested to enhance DICER activity. Abnormal levels of miRNA are a common molecular mechanism underlying multiple forms of familial and sporadic human ALS and enhancement of DICER activity was found to be beneficial *in vivo, see* Emde A, Eitan C, Liou LL, et al. Dysregulated miRNA biogenesis downstream of cellular stress and ALS-causing mutations: a new mechanism for ALS. The EMBO journal 2015;34:2633-51*.*

Ciprofloxacin was introduced to the swimming water of the SOD1 mutant larvae in three final concentrations- 1µM, 10µM and 100µM. The experiment was conducted on the background of 0.1% DMSO in all samples (including the control sample). In all Ciprofloxacin doses, no drug induced effects in morphology or mortality were observed.

Summary of section IV: The normalized values for each experiment comparing percentage of distance moved of all distinct Ciprofloxacin- treated populations were compared to the non-treated SOD1 mutant larvae. Two main observations can be made. First, ciprofloxacin treatment, even in high doses, was not toxic to SOD1 mutant larvae. Second, it seems that under these study conditions, the 100uM Ciprofloxacin concentration was the most potent in affecting locomotor activity of the ALS model.

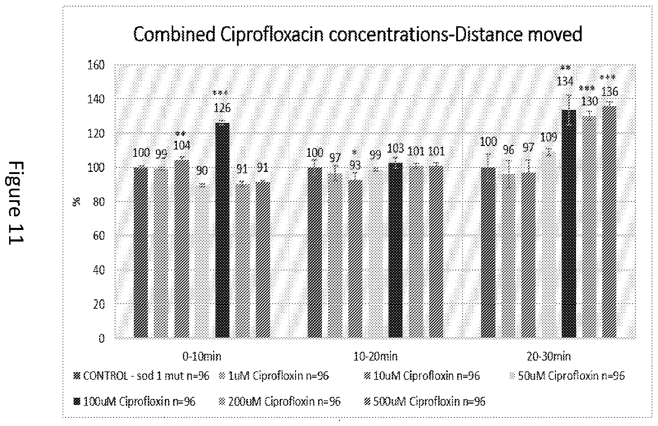

Summary of all normalized values representing the percentage of distance moved of Ciprofloxacin- treated vs non- treated SOD1 larvae refining the three phases of the experiment showed unexpected results **(****Figure 11****).**

Treatment with 1, 10, 50, 100, 200 and 500µM Ciprofloxacin had nearly no effect on locomotor activity of the SOD1 mutant larvae under dark conditions **(****Figure 11****).** In the second phase of light, the phase of recovery from stress, treatment with 50, 100, 200 and 500µM Ciprofloxacin gave a dose dependent curve that eventually ended in a plateau. ANOVA and t-test static studies showed no statistically significant change between the 200 and 500µM Ciprofloxacin treatments (the statistics presented is significance from non- treated).

During the period of spontaneous swimming, only a dose of 100uM Ciprofloxacin caused elevated swimming behavior **(****Figure 11****).** Two possible explanations can underlie the fact that 200 and 500µM Ciprofloxacin treatment did not cause elevation in spontaneous swimming of SOD1 mutants: subtle burden on swimming ability due to high Ciprofloxacin concentrations or, although less reasonable, elevated levels of NaCl salt in the swimming media (or both). To adjust pH levels to that of the control group (no Ciprofloxacin), NaOH was added to a final concentration of 0.06mM in the 50 and 100µM Ciprofloxacin treatments, 0.135mM and 0.32mM NaOH in the 200µM and 500µM Ciprofloxacin treatments, respectively.

**Figure 11** discloses a summary of locomotor activity of SOD1 mutants treated with Ciprofloxacin distinct concentrations during all 3 phases of the experiment

### V. Toxicity and efficacy of treatment with Celecoxib and Ciprofloxacin combinations in ALS (SOD1 mut) model

Combining cox2 inhibitor Celecoxib that was suggested to have a role as neuro-inflammatory modulator in maintaining macrophages in their neuroprotective state²³ with Ciprofloxacin which has been previously suggested to enhance DICER activity to attenuate miRNA abnormal levels, *see* Emde A, Eitan C, Liou LL, et al. Dysregulated miRNA biogenesis downstream of cellular stress and ALS-causing mutations: a new mechanism for ALS. The EMBO journal 2015;34:2633-51*,* was of interest, as they both tackle two main mechanisms underlying ALS. Furthermore, synergistic activity of both agents has been demonstrated, see Dey R, Sultana S, Bishayi B. Combination treatment of celecoxib and ciprofloxacin attenuates live S. aureus induced oxidative damage and inflammation in murine microglia via regulation of cytokine balance. J Neuroimmunol 2018;316:23-39*.*

Due to toxic limitation in the use of Celecoxib, we decided to use 1, 4 and 10µM Celecoxib in combination with the most potent concentration of Ciprofloxacin (100µM). The experiment was conducted on the background of 0.1% DMSO in all samples.

In all combinations used (100µM Cipro + 1µM Celecoxib; 100uM Cipro + 4µM Celecoxib; 100µM Cipro + 10µM Celecoxib), no cardiovascular system abnormalities (heart rate, morphology, hemorrhage and edema) or mortality were observed. Treating SOD1 mutant larvae with the highest concentration of Celecoxib with Ciprofloxacin (100µM Cipro + 10µM Celecoxib) had visible effect on their swimming ability under the microscope. The larvae swam shorter distances and fell on their side between movements. No obvious abnormalities in movement were evident in SOD1 mutant larvae treated with the combinations containing lower Celecoxib doses (100µM Cipro + lµM Celecoxib; 100uM Cipro + 4µM Celecoxib).

The averaged distance that treated larvae moved per time bin of 1 minute was analyzed using the EthoVision software.

The abnormal movement observed under the microscope in the 100+10 mix (100uM Cipro + 10µM Celecoxib)-treated SOD1 mutant larvae was evident; the larvae swam shorter distances during the spontaneous swimming and light / dark challenge periods. The mix containing the lowest Celecoxib concentration 100+1 mix (100uM Cipro + 1µM Celecoxib) gave substantial improvement in SOD1 mutant swimming ability during all behavioral response profiles .

Next, the distance in mm all larvae from the same treatment swam in time bin of 1 minute was averaged for the whole period of time to get a relative number compared to the non- treated SOD1 mutant swimming behavior **(****Figure 4). Figure 4** depicts ciprofloxacin and celecoxib combinations- treated SOD1 mutants' locomotor activity compared to non- treated SOD1 mutants during the whole period of measurement.

As a whole, the SOD1 mutants treated with the 100+1 mix showed dramatic and significant improvement in swimming ability (28.8%, **Figure 4****).** SOD1 mutants treated with the 100+4 mix showed significant improvement in their swimming ability, albeit lower than the 10+1 mix (14.9%). SOD1 mutants treated with 100+10 mix showed significant reduction in their swimming behavior (decrease of 20.7%, **Figure 4****).**

The distance moved per each part of the experiment was calculated and compared to the non-treated SOD1 mutant swimming behavior **(****Figure 3). Figure 3** depicts Ciprofloxacin and Celecoxib combinations - treated SOD1 mutants' locomotor activity compared to non- treated SOD1 mutants in all 3 phases of the experiment. In all three phases of the experiment, efficacy of the distinct combinations was dose dependent, with higher potency as Celecoxib doses decreased in the mix **(****Figure 3****).** SOD1 mutants treated with the 100+1 mix showed outstanding improvement in their swimming ability during spontaneous swimming (25.3%), light/ dark challenge (11.8%) and most dramatically during recovery from challenge (72.2% !).

SOD1 mutants treated with the 100+4 mix showed less but yet significant increase in their swimming ability during spontaneous swimming (14.5%) and recovery from challenge (42.8%).Treatment with 100+10 mix caused statistically significant reduction in locomotor activity of the SOD1 mutant larvae during spontaneous swimming (31.4% decrease) and light/ dark challenge (29.4% decrease). During recovery from challenge, increase of 17.7% was evident even in the 100+10 mix **(****Figure 3****).**

As the combination of 100µM Cipro and lµM Celecoxib gave such substantial improvement in SOD1 mutant swimming ability, it was decided to further check its effect on SOD1 mutant larvae using morphological assays.

### VI: Morphological analysis of ventral motor neuron following treatment with Celecoxib and Ciprofloxacin combination in ALS (SOD1 mut) model

ALS zebrafish models mutated in distinct ALS- associated genes such as TDP-43, FUS, C9orf72, Scistml, EPHA4 and SOD1, exhibit motor axons phenotype consisting of disorganized, excessively branched motor neuronal axons as well as swimming deficits , see Kabashi et al., PLoS genetics 2011;7:e1002214*; .*Lemmens et al., Human molecular genetics 2007;16:2359-65*;*Lattante et al., Human molecular genetics 2015;24:1682-90*;* Sorana et al., Annals of neurology 2013; 74:180-7*;* Van Hoecke et al., Nature medicine 2012;18:1418*;* Sakowski SA, Lunn JS, Busta AS, et al. Neuromuscular effects of G93A-SOD1 expression in zebrafish. Molecular neurodegeneration 2012;7:44*; .*Armstrong et al., PloS one 2016;11:e0150188*.*

We used immunostaining, apotome microscopy and Imaris image analysis software to characterize motor neurons morphology in WT, non- treated SOD1 G93R mutants and SOD1 G93R mutants treated with the 100µM Cipro + 1µM Celecoxib combination **(****Figure 14****).**

WT zebrafish predominantly exhibited normal motor neurons, with long and moderately branched axons **(****Figure 14****,** left panel).

The SOD1 G93R mutant larvae, all originating from the same laying batch, were divided into two groups, treated (half with DMSO only and half with the mix), stained and imaged. The ALS model SOD1 mutant control group (untreated, 0.1% DMSO) showed severe axonopathy with highly complex branched fibers **(****Figure 14****,** middle panel). Remarkably, the half of SOD1 mutant larvae that were treated with the combination of 1 µM Celecoxib and 100µM Ciprofloxacin showed significant recovery of the mutant morphology, and gained nearly normal axon morphology **(****Figure 14****,** left panel).

In summary, **Figure 14** discloses that administration of Ciprofloxacin and Celecoxib recovered axonopathy of SOD 1 mutant fish. Morphological analysis of individual motor neurons in the trunk of zebrafish larva; segments S10- S12 were used for analysis. Left panel- WT, middle panel- non-treated SOD1 mutant fish, left panel- 1µM Celecoxib + 100µM Ciprofloxacin- treated SOD1 mutant fish. Upper panel- 3D reconstructed apotome z-stack images of branching motor neurons in the trunk of 6dpf zebrafish larvae immunostained with anti-acetylated tubulin antibodies. Middle and lower panels- The backbone (colored processes) of motor neurons traced with the Filaments analysis of Imaris software (Bitplane; in white- single motor neuron backbone). n=15; control and treated SOD1 mutants and n=11; WT fish.

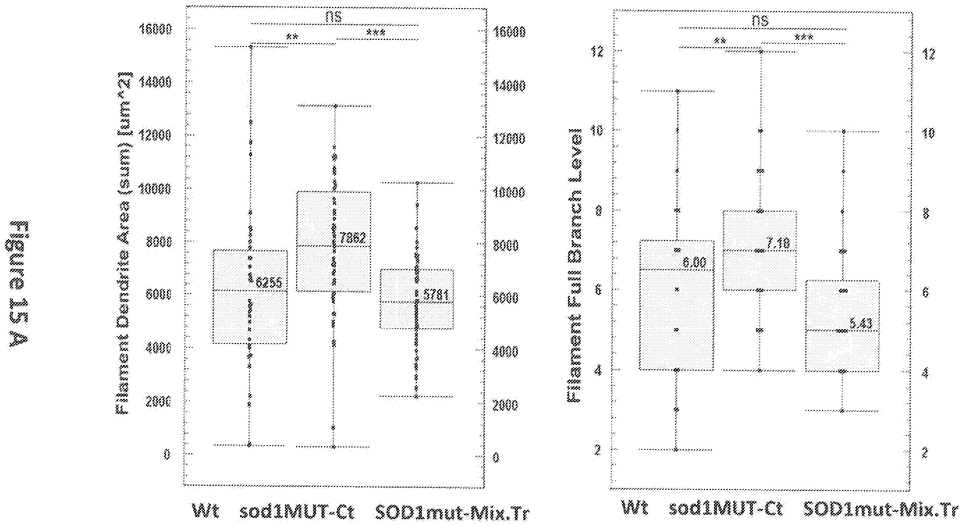

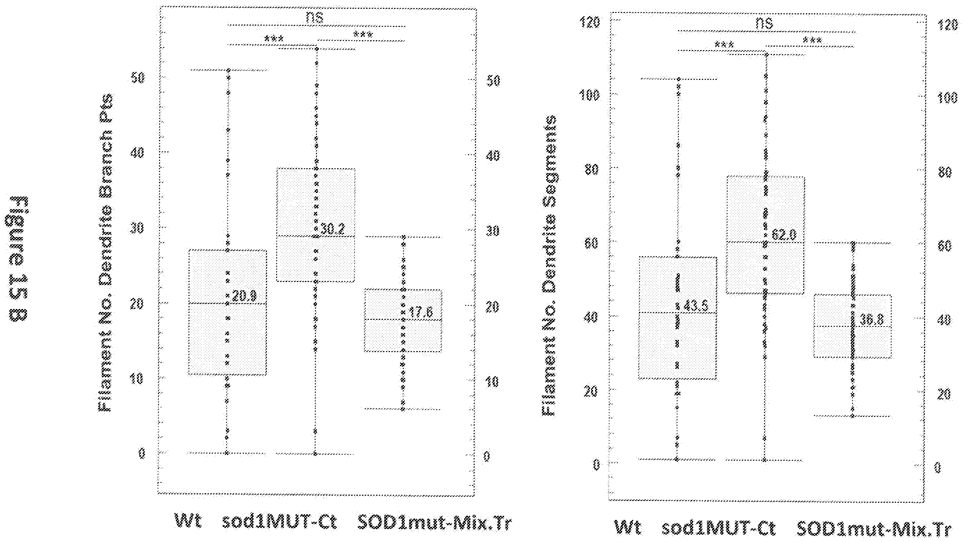

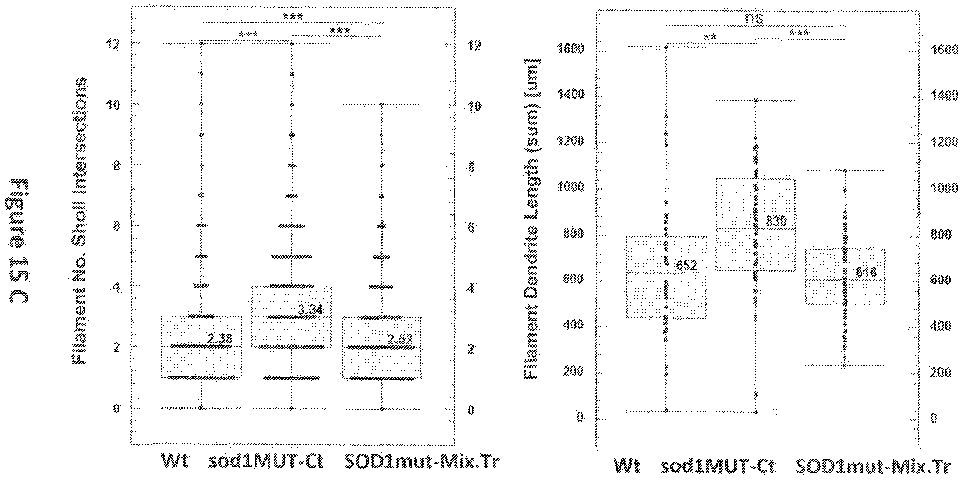

High- resolution image analyses were conducted using the Imaris software to compare distinct parameters of axons morphology **(****Figure 15****).** All examined parameters showed reduction in axonopathy in the 1µM Celecoxib and 100µM Ciprofloxacin- treated SOD1 mutant fish compared to non- treated SOD1 mutants, including area, branching level, branch points (junctions), segments, length and their spreading in Sholl analysis and showed significant recovery towards the wild type axon morphology **(****Figure 15****).**

Combination of Ciprofloxacin and Celecoxib caused a nearly full recovery of motor neurons axonopathy of SOD1 mutant fish. In summary, **Figure 15** depicts that combination of ciprofloxacin and celecoxib caused a nearly full recovery of motor neurons axonopathy of SOD1 mutant fish. Aspects of length and branching of motor neurons axonal projections were calculated using the Imaris software (Bitplane) and are plotted in the graphs.

### VII. Comparing efficacy of treatment with Enoxacin vs Ciprofloxacin in ALS (SOD1 mut) model

Enoxacin is a small molecule from the quinolone family that enhances siRNA-mediated mRNA degradation and promotes the biogenesis of endogenous miRNAs, *see* Melo et al., Proceedings of the National Academy of Sciences of the United States of America 2011;108:4394-9*., and* Shan et al. A small molecule enhances RNA interference and promotes microRNA processing. Nature biotechnology 2008;26:933-40*.* Both *in vitro* and *in vivo* ALS models showed benefit when treated with Enoxacin, see Emde et al., The EMBO journal 2015;34:2633-51*.,* and Shan et al., Nature biotechnology 2008;26:933-40*.* Ciprofloxacin, another quinolone family member, is commercially available and was shown to have substantial RNAi-enhancing activity. Its effect was slightly lesser than Enoxacin in *in vitro* RNAi reporter assays, see *Shan ibid.* In this study, Ciprofloxacin showed a significant improvement in the swimming activity of SOD1 mutant larvae **(****Figure 10****).**

To examine the hypothesis that Ciprofloxacin, like Enoxacin, may contribute to ALS model *in vivo,* we set out to compare Enoxacin and Ciprofloxacin effect on motor ability of the SOD1 G93R zebrafish mutant.

Enoxacin was introduced to the swimming water of the SOD1 mutant larvae in two final concentrations- 10µM and 100µM. The experiment was conducted on the background of 0.1% DMSO in all samples.

In both Enoxacin doses, no drug induced effects in morphology or mortality were observed. The averaged distance that treated larvae moved per time bin of 1 minute was analyzed. Treatment with 100µM Enoxacin caused increase in locomotor activity of ALS larvae. Dose of 10µM Ciprofloxacin did not significantly affect locomotor activity of the treated mutant larvae throughout the experiment. A very similar dose effect was seen in Ciprofloxacin- treated larvae.

During the whole experiment, the distance in mm that the 10µM enoxacin- treated SOD1 mutant larvae swam was not changed compared to the non- treated SOD1 mutant larvae. The SOD1 mutants treated with 100µM enoxacin showed substantial increase in their swimming behavior (19.9%). Similarly, during the whole experiment, the distance in mm that the 10µM Ciprofloxacin - treated SOD1 mutant larvae swam was not changed compared to the non- treated SOD1 mutant larvae, while the SOD1 mutants treated with 100µM Ciprofloxacin showed substantial increase in their swimming behavior (17.8%).

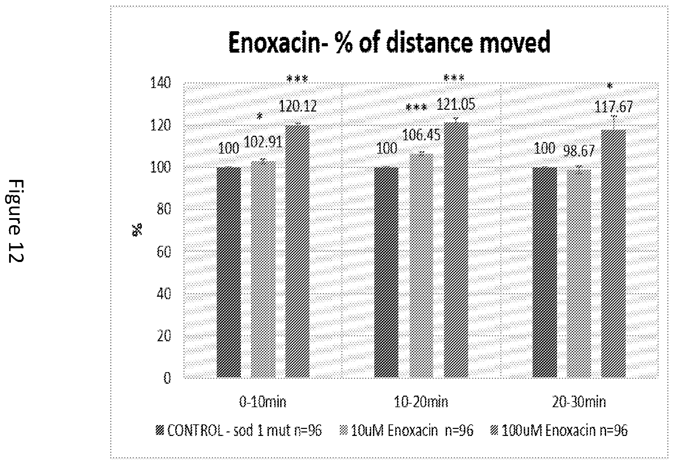

The distance moved per each part of the experiment was calculated and compared to the non-treated SOD1 mutant swimming behavior **(****Figure 12****).** Treatment with 10µM enoxacin had nearly no effect on locomotor activity of the SOD1 mutant larvae during spontaneous swimming, light/ dark challenge and recovery from second challenge. Treatment with 100µM enoxacin showed significant increase in locomotor activity in the phase of spontaneous swimming (20.1%), light/ dark challenge (21%) and following recovery from challenge (17.6%, **Figure 12****).**

**Figure 12** depicts enoxacin- treated SOD1 mutants' locomotor activity compared to non- treated SOD1 mutants in all 3 phases of the experiment.

Correspondingly, treatment with 10µM Ciprofloxacin had nearly no effect on locomotor activity of the SOD1 mutant larvae during all three experimental phases **(****Figure 10****).** Treatment with 100µM Ciprofloxacin showed significant increase in locomotor activity in the phase of spontaneous swimming (26.2%) and following recovery from challenge (33.5%).

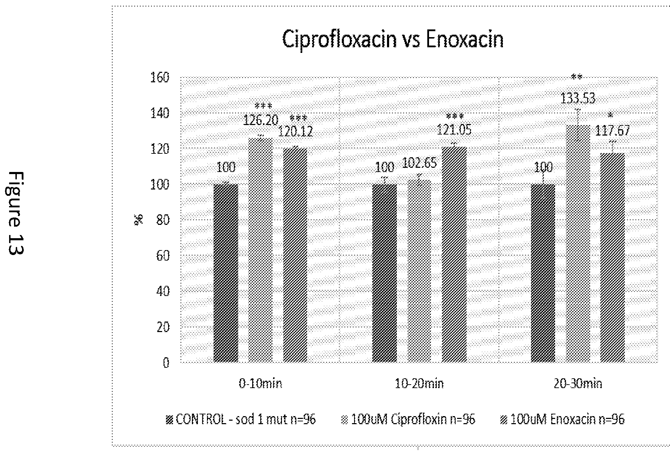

To conclude this part, comparisons between treatments with 100µM ciprofloxacin and 100µM enoxacin are presented in **Figure 13. Figure 13** depicts normalized enoxacin vs Ciprofloxacin-treated SOD1 mutants' locomotor activity compared to non- treated SOD1 mutants in all 3 phases of the experiment.

First, both enoxacin and Ciprofloxacin caused similar overall increase in locomotor activity of SOD1 mutant larvae in the same range of concentrations. Second, treating SOD1 mutants with Ciprofloxacin caused elevated locomotor activity compared to Enoxacin treatment during both spontaneous swimming and recovery from challenge **(****Figure 13****).**

### EXAMPLE 3

The combination of ciprofloxacin and celecoxib is formulated to either oral administration, intravenous administration or topical administration.

The formulations of the present invention comprise *inter alia,* in a non-limiting matter, additional ingredients or pharmaceutical excipients to further develop a formula to have a desired concentration, effective doses, dosing regiments and treatment times. These ingredients include, *inter alia,* solubilizers, stabilizers, buffers, tonicity modifiers, bulking agents, viscosity enhancers/reducers, surfactants, chelating agents, and adjuvants.

Oral administration Oral drugs are taken as tablets or capsules.

Tablets: The dissolution of the tablet can be affected significantly by particle size and crystal form. The dissolution time can be modified for a rapid effect (fast dissolution) or for sustained release, (slow dissolution rates which prolong the duration of action or avoid initial high plasma levels).

Capsules: A capsule is a gelatinous envelope enclosing the active substance. Capsules can be designed to remain intact for some hours after ingestion in order to delay absorption. They may also contain a mixture of slow- and fast-release particles to produce rapid and sustained absorption in the same dose.

Oral sustained release: Oral sustained release in capsules or tablets is achieved, in a non-limiting matter, by embedding the active ingredient in an insoluble porous matrix, such that the dissolving drug must make its way out of the matrix before it can be absorbed, sustained release formulations in which the matrix swells to form a gel through which the drug exits, or by an osmotic controlled-release oral delivery system, where the active compound is encased in a water-permeable membrane with a laser drilled hole at one end. As water passes through the membrane the drug is pushed out through the hole and into the digestive tract where it can be absorbed.

Solutions: Pharmaceutical solutions are extensively used as dosage forms for the oral administration of therapeutic agents. Pharmaceutical solutions defined as liquid preparations in which the therapeutic agent and the various excipients are dissolved in the chosen solvent system. Pharmaceutical solutions are homogeneous, i.e. the therapeutic agent(s) and excipients are dissolved in the vehicle

Parenteral administration: Parenteral administration is performed using intravenous, subcutaneous, intramuscular, and intra-articular administration. The drug is stored in liquid or if unstable, lyophilized form.

Topical administration: Topical formulations comprise *inter alia* cream, ointment, paste, lotion or gel.

Transdermal delivery: Transdermal delivery is achieved, for example, by transdermal patches.

Alternative routes of administration are suppository, intraventricular, intramuscular, inhalational, aerosol, and sublingual.

### EXAMPLE 4

The composition of the current invention is used in the aforementioned ratios of combinations of: Weight /weight daily human doses.

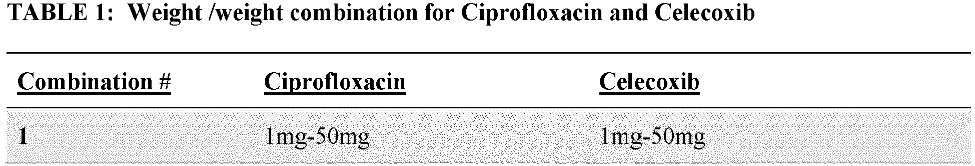

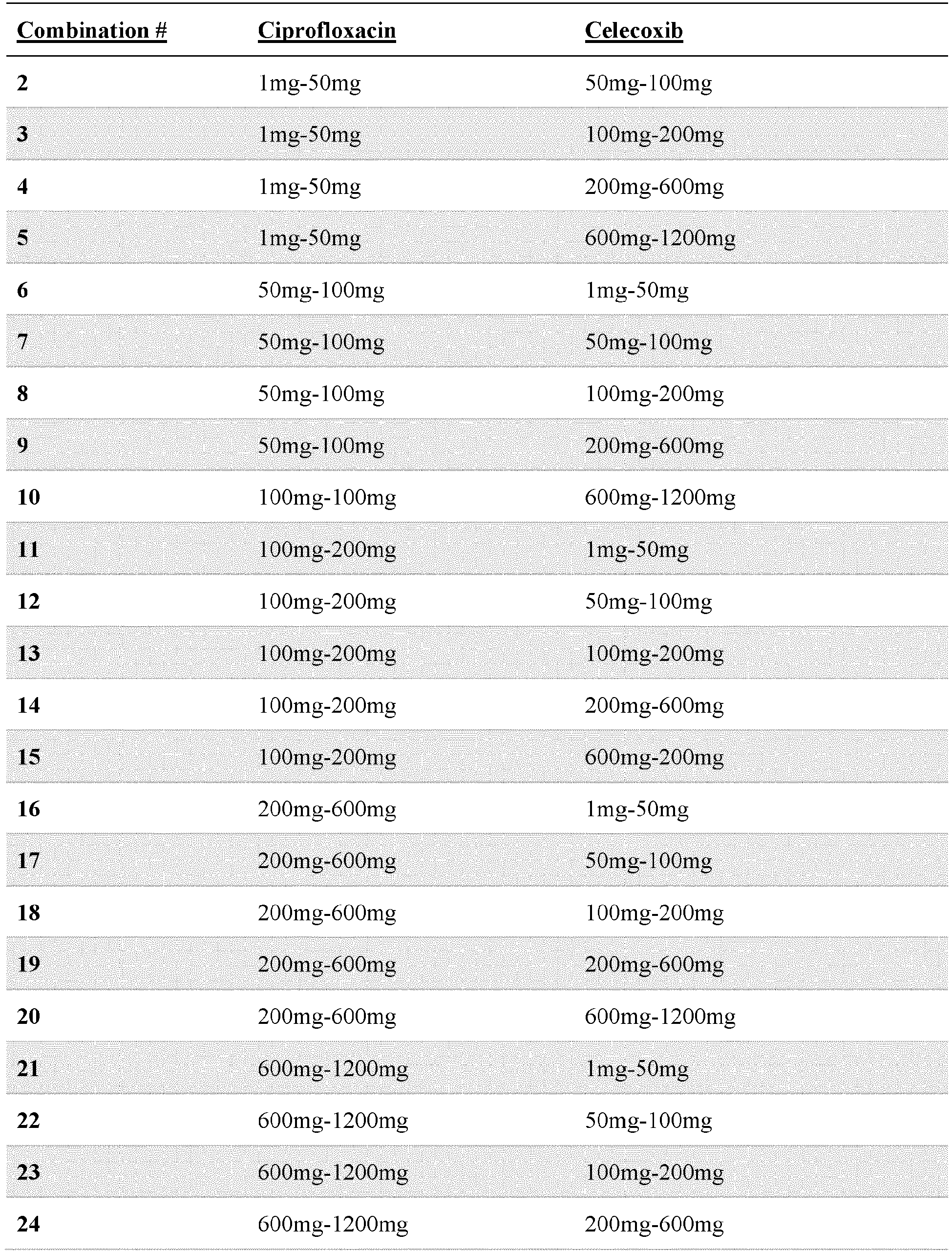

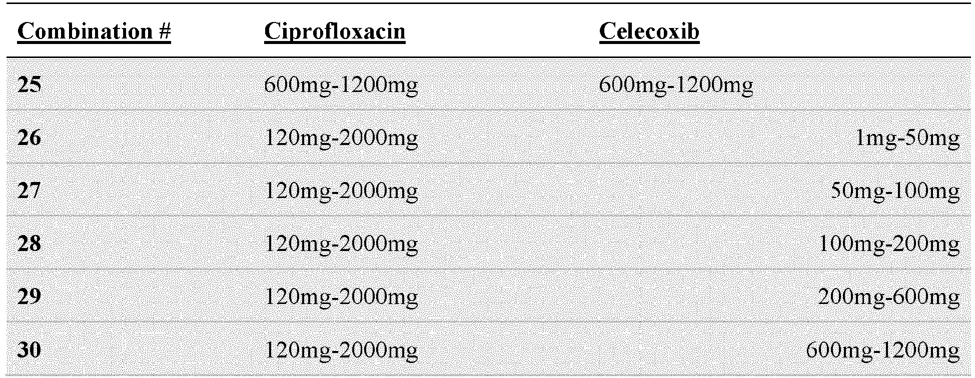

### EXAMPLE 5

A synopsis of a clinical trial for evaluation the therapeutic effect of a combination of celecoxib and ciprofloxacin.

As used herein after, the term **"Prime C"** generally refers hereinafter to a composition comprising a combination of celecoxib and ciprofloxacin.

**TABLE 2 synopsis for a clinical trial**

| **Title** | A Phase 2, Multi-Center, Double-Blind, Randomized, Placebo-Controlled Study to Evaluate the Safety, Tolerability, and Efficacy of Prime_C in Patients with Amyotrophic Lateral Sclerosis (ALS) | |
|---|---|---|
| **Indication** | Amyotrophic Lateral Sclerosis (ALS) | |
| **Study Population** | Patients with a diagnosis of ALS for less than 24 months | |
| **Primary Objective** | To assess the effect of Prime_C versus placebo on respiratory function in patients with ALS | |
| **Primary Endpoint** | The change from baseline to Visit Week 24 in EEVI 150 Hz phase of the fastest changing muscle in active treatment vs placebo at 24 weeks | |
| **Secondary Objectives** | 1. | To assess the effect of Prime_C versus placebo on the HHDO time to failure endpoint using HHD |
| | 2. | To assess the effect of Prime_C versus placebo on vital capacity as assessed at home twice weekly |
| | 3. | To assess the effect of Prime_C versus placebo on ALSFRS-R as assessed at home twice weekly |
| | 4. | To assess the effect of Prime_C versus placebo on vital capacity and ALSFRS-R at baseline compared to week 24 |
| | 5. | To assess safety and tolerability of Prime_C 1 in patients with ALS |
| | 6. | To assess changes in pNFH in csf and in blood from baseline to week 24 in patients on Prime_C vs placebo |
| | 7. | To assess changes in miRNAs in csf and in blood from baseline to week 24 in patients on Prime_C vs placebo |
| **Secondary Endpoints** | 1. | Time to first zero muscle strength (HHDO) in Prime_C 1 patients compared to placebo |
| | 2. | Change in slope from baseline to Visit Week 24 in vital capacity measured at home twice weekly in patients on Prime_C 1 compared to placebo |
| | 3. | Change in slope from baseline to Visit Week 24 in ALSFRS-R measured at home twice weekly in patients on Prime_C 1 compared to placebo |
| | 4. | Change in slope from baseline to Visit Week 24 in the ALS Functional Rating Scale - Revised (ALSFRS-R) and vital capacity measured at study visits in patients on Prime_C compared to placebo |
| | 5. | Subject incidence of adverse events in patients on Prime_C compared to placebo |
| | 6. | Change in pNFH in csf and in blood from baseline to week 24 in patients on Prime_C 1 vs placebo |
| | 7. | Change in miRNAs in csf and in blood from baseline to week 24 in patients on Prime_C 1 vs placebo |
| **Safety Measurements** | 1. | Adverse events |
| | 2. | Vital signs |
| | 3. | Clinical laboratory parameters |
| | 4. | ECG parameters |
| | 5. | Physical and neurological examinations |
| | 6. | Suicidality assessment |
| **Study Overview** | This is a Phase 2, double-blind, randomized, placebo-controlled, multiple dose study of Prime_C in patients with ALS. | |
| | Randomization will be 1:1, Prime_C vs placebo. Within each cohort, randomization will be stratified by riluzole and edaravone use. The screening and qualification period for the study will be no more than 14 days in duration. Once patients have completed screening and are considered eligible for the study, they will be randomized as described above, stratified by riluzole use. | |
| | There will be a total of 7 study visits for patients in each cohort: | |
| | | • Screening |
| | | • Start of Dosing (Day 1) |
| | | • Week 8 (Day 57) |
| | | • Week 16 (Day 113) |
| | | • Week 24 (Day 169) |
| | | • Telephone follow-up Visit (4 weeks after last dose of study drug) |
| **Number of Patients** | Approximately 200 patients with ALS will be screened in order to enroll at least 150 patients with ALS. | |
| **Estimated Study Duration** | Individual patient participation will last approximately 26 weeks, divided as follows: | |
| | | 1. Screening and qualification period: up to 2 weeks |
| | | 2. Double-blind treatment, safety, and efficacy assessment period for 24 weeks of dosing |
| | Total study duration is anticipated to be up to 24 months, with 18 months allocated for study start-up and patient recruitment. | |
| **Study Assessments** | | • EIM |
| | | • HHD muscle testing |
| | | • VC |
| | | • ALSFRS-R |
| | | • SVC |

| | | |
|---|---|---|
| EIM - Electric Impedance Myography, HHD - Hand-Held Dynamometers, VC - Vital Capacity, ALSFRS-r - ALS Functional Rating Scale revised, and SVC - Slow Vital Capacity. | | |

## Claims

1. A composition for use in treating amyotrophic lateral sclerosis (ALS) in a mammalian subject, wherein said composition comprises a synergistic combination of celecoxib and ciprofloxacin, wherein the celecoxib to ciprofloxacin ratio ranges from 1:10 to 1:200 (wt/wt).

2. The composition for use according to claim 1, wherein said ciprofloxacin is ciprofloxacin-HCl.

3. The composition for use according to claim 1 or claim 2, wherein the celecoxib to ciprofloxacin ratio is 1:10 (wt/wt).

4. The composition for use according to any one of claims 1 to 3, wherein said composition is configured to be administrable in a manner selected from a group consisting of a tablet, a capsule, a pill, lyophilized powder, emulsion, granulated powder, cream, ointment, paste, lotion, gel, liquid, a solution, a patch and any combination thereof.

5. The composition for use according to any one of claims 1 to 4, wherein said composition is configured to be administrable in a manner selected from a group consisting of fast release, slow release, sustained release, controlled release and any combination thereof.

6. The composition for use according to any one of claims 1 to 5, wherein said composition additionally comprises ingredients selected from a group consisting of solubilizers, stabilizers, buffers, tonicity modifiers, bulking agents, viscosity enhancers/reducers, surfactants, chelating agents, adjuvants and any combination thereof.

7. A kit comprising celecoxib and ciprofloxacin for use in a synergistic treatment of amyotrophic lateral sclerosis (ALS) in a mammalian subject, wherein the celecoxib to ciprofloxacin ratio ranges from 1:10 to 1:200 (wt/wt).

8. The composition for use according to any one of claims 1 to 6, wherein said composition is configured to be administrable in a manner selected from a group consisting of oral, topical, dermal, transdermal, intravenous, subcutaneous, intramuscular, intra-articular, suppository, intraventricular, inhalational, aerosol, sublingual and any combination thereof.

9. The composition for use according to any one of claims 1 to 6, wherein said composition is configured to be administrable in a manner selected from a group consisting of one at a time, non-simultaneous, sequentially and concomitantly.

10. The composition for use according to any one of claims 1 to 6, wherein said composition is configured to be administrable in a manner selected from a group consisting of single dose, single daily dose, twice daily dose, continuous dose, infusion, and any combination thereof.

## Patentansprüche

1. Zusammensetzung zur Verwendung bei der Behandlung von amyotropher Lateralsklerose (ALS) bei einem Säugetier, wobei die Zusammensetzung eine synergistische Kombination von Celecoxib und Ciprofloxacin umfasst, wobei das Verhältnis von Celecoxib zu Ciprofloxacin im Bereich von 1:10 bis 1:200 (Gew./Gew.) liegt.

2. Zusammensetzung zur Verwendung nach Anspruch 1, wobei das Ciprofloxacin Ciprofloxacin-HCl ist.

3. Zusammensetzung zur Verwendung nach Anspruch 1 oder Anspruch 2, wobei das Verhältnis von Celecoxib zu Ciprofloxacin 1:10 (Gew./Gew.) beträgt.

4. Zusammensetzung zur Verwendung nach einem der Ansprüche 1 bis 3, wobei die Zusammensetzung so konfiguriert ist, dass sie in einer Weise verabreicht werden kann, die aus einer Gruppe ausgewählt ist, die aus einer Tablette, Kapsel, Pille, einem lyophilisierten Pulver, einer Emulsion, einem granulierten Pulver, einer Creme, Salbe, Paste, Lotion, einem Gel, einer Flüssigkeit, Lösung, einem Pflaster und einer beliebigen Kombination davon besteht.

5. Zusammensetzung zur Verwendung nach einem der Ansprüche 1 bis 4, wobei die Zusammensetzung so konfiguriert ist, dass sie in einer Weise verabreicht werden kann, die aus einer Gruppe ausgewählt ist, die aus einer raschen Freisetzung, einer langsamen Freisetzung, einer verzögerten Freisetzung, einer kontrollierten Freisetzung und einer beliebigen Kombination davon besteht.

6. Zusammensetzung zur Verwendung nach einem der Ansprüche 1 bis 5, wobei die Zusammensetzung zusätzlich Bestandteile umfasst, die aus einer Gruppe ausgewählt sind, die aus Lösungsvermittlern, Stabilisatoren, Puffern, Tonizitätsmodifizierern, Füllstoffen, Viskositätsverstärkern/- reduzierern, Tensiden, Chelatbildnern, Adjuvantien und einer beliebigen Kombination davon besteht.

7. Kit, umfassend Celecoxib und Ciprofloxacin zur Verwendung bei einer synergistischen Behandlung von amyotropher Lateralsklerose (ALS) bei einem Säugetier, wobei das Verhältnis von Celecoxib zu Ciprofloxacin im Bereich von 1:10 bis 1:200 (Gew./Gew.) liegt.

8. Zusammensetzung zur Verwendung nach einem der Ansprüche 1 bis 6, wobei die Zusammensetzung so konfiguriert ist, dass sie in einer Weise verabreicht werden kann, die aus einer Gruppe ausgewählt ist, die aus oral, topisch, dermal, transdermal, intravenös, subkutan, intramuskulär, intraartikulär, als Zäpfchen, intraventrikulär, inhalativ, als Aerosol, sublingual und einer beliebigen Kombination davon besteht.

9. Zusammensetzung zur Verwendung nach einem der Ansprüche 1 bis 6, wobei die Zusammensetzung so konfiguriert ist, dass sie in einer Weise verabreicht werden kann, die aus einer Gruppe ausgewählt ist, die aus einem von einzeln, nicht gleichzeitig, nacheinander und begleitend besteht.

10. Zusammensetzung zur Verwendung nach einem der Ansprüche 1 bis 6, wobei die Zusammensetzung so konfiguriert ist, dass sie in einer Weise verabreicht werden kann, die aus einer Gruppe ausgewählt ist, die aus einer Einzeldosis, einer Dosis einmal täglich, einer Dosis zweimal täglich, einer kontinuierlichen Dosis, einer Infusion und einer beliebigen Kombination davon besteht.

## Revendications

1. Composition destinée à être utilisée dans le traitement de la sclérose latérale amyotrophique (SLA) chez un sujet mammifère, dans laquelle ladite composition comprend une combinaison synergique de célécoxib et de ciprofloxacine, dans laquelle le rapport célécoxib sur ciprofloxacine est compris entre 1:10 et 1:200 (p/p).

2. Composition destinée à être utilisée selon la revendication 1, dans laquelle ladite ciprofloxacine est la ciprofloxacine-HCI.

3. Composition destinée à être utilisée selon la revendication 1 ou la revendication 2, dans laquelle le rapport célécoxib sur ciprofloxacine est de 1:10 (p/p).

4. Composition destinée à être utilisée selon l'une quelconque des revendications 1 à 3, dans laquelle ladite composition est configurée pour être administrable d'une manière choisie dans un groupe constitué d'un comprimé, d'une capsule, d'une pilule, d'une poudre lyophilisée, d'une émulsion, d'une poudre granulée, d'une crème, d'une pommade, d'une pâte, d'une lotion, d'un gel, d'un liquide, d'une solution, d'un patch et toute combinaison de ceux-ci.

5. Composition destinée à être utilisée selon l'une quelconque des revendications 1 à 4, dans laquelle ladite composition est configurée pour être administrable d'une manière choisie dans un groupe constitué d'une libération rapide, d'une libération lente, d'une libération prolongée, d'une libération contrôlée et toute combinaison de celles-ci.

6. Composition destinée à être utilisée selon l'une quelconque des revendications 1 à 5, dans laquelle ladite composition comprend en outre des ingrédients choisis dans un groupe constitué de solubilisants, de stabilisants, de tampons, de modificateurs de tonicité, d'agents de charge, d'exhausteurs/réducteurs de viscosité, de tensioactifs, d'agents chélatants, d'adjuvants et toute combinaison de ceux-ci.

7. Kit comprenant du célécoxib et de la ciprofloxacine destiné à être utilisé dans un traitement synergique de la sclérose latérale amyotrophique (SLA) chez un sujet mammifère, dans lequel le rapport célécoxib sur ciprofloxacine est compris entre 1:10 et 1:200 (p/p).

8. Composition destinée à être utilisée selon l'une quelconque des revendications 1 à 6, dans laquelle ladite composition est configurée pour être administrable d'une manière choisie dans un groupe constitué de la voie orale, topique, dermique, transdermique, intraveineuse, sous-cutanée, intramusculaire, intra-articulaire, suppositoire, intraventriculaire, par inhalation, en aérosol, sublinguale et toute combinaison de ceux-ci.

9. Composition destinée à être utilisée selon l'une quelconque des revendications 1 à 6, dans laquelle ladite composition est configurée pour être administrable d'une manière choisie dans un groupe constitué de l'une à la fois, non simultanée, séquentielle et concomitante.

10. Composition à utiliser selon l'une quelconque des revendications 1 à 6, dans laquelle ladite composition est configurée pour être administrable d'une manière choisie dans un groupe constitué d'une dose unique, d'une dose quotidienne unique, d'une dose biquotidienne, d'une dose continue, d'une perfusion et toute combinaison de celles-ci.
